(19) Europäisches Patentamt
European Patent Office
Office européen des brevets

(11) **EP 3 376 462 A1**

(12) **EUROPEAN PATENT APPLICATION**
published in accordance with Art. 153(4) EPC

(43) Date of publication:
**19.09.2018 Bulletin 2018/38**

(51) Int Cl.:
***G06Q 50/10*** *(2012.01)*

(21) Application number: **15908345.0**

(86) International application number:
**PCT/JP2015/082050**

(22) Date of filing: **13.11.2015**

(87) International publication number:
**WO 2017/081829 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **MORI, Tatsuya**
**Kawasaki-shi**
**Kanagawa 211-8588 (JP)**

• **MAEDA, Kazuho**
**Kawasaki-shi**
**Kanagawa 211-8588 (JP)**
• **INOMATA, Akihiro**
**Hayes**
**Middlesex**
**UB4 8FE (GB)**

(74) Representative: **Haseltine Lake LLP**
**Lincoln House, 5th Floor**
**300 High Holborn**
**London WC1V 7JH (GB)**

(54) **BEHAVIOR DETECTION DEVICE, BEHAVIOR DETECTION METHOD, AND BEHAVIOR DETECTION PROGRAM**

(57) An information processing device (100) executes processes of acquiring sensor data from one or more sensors; calculating a first characteristic amount serving as an index of characteristic of biological reaction to a behavior to be sensed from the sensor data; calculating a probability of occurrence of the behavior as a first behavior occurrence probability from the first characteristic amount; calculating a second characteristic amount serving as an index of characteristic of a habit relating to the behavior to be sensed from the sensor data; calculating a probability of occurrence of the behavior as a second behavior occurrence probability from the second characteristic amount; and determining whether the behavior is performed based on the first behavior occurrence probability and the second behavior occurrence probability.

FIG.1

Printed by Jouve, 75001 PARIS (FR)

**Description**

[Technical Field]

The present invention relates to a behavior sensing device, a behavior sensing method and a behavior sensing program.

[Background Art]

With the aging of society and an increase in medical expenses, healthcare is drawing attention. Furthermore, wearable sensors and Internet of Things (IoT) devices have become popular and an environment enabling acquisition of various types of information from various devices is being created. Against the background, in order to grasp the lifestyle and health condition by behavior sensing using various types of devices, technologies to sense behaviors relating to healthcare by machine learning have been proposed.

[Citation List]

[Patent Citation]

Patent Document

Patent Document 1: Japanese Laid-open Patent Publication No. 2013-109623

[Summary of Invention]

[Technical Problem]

With the above-described technology, however, a failure to sense a behavior or false sensing of a behavior may occur due to the following reasons.
The above-described technology reduces the effect of differences among individuals by learning a relationship between a behavior A to be sensed and a usual behavior and a situation that relate to the behavior A, that is, a behavior habit, to seek to improve accuracy. In a case where behavior sensing is performed by machine learning, when a behavior conflicting a behavior habit is performed, for example, when the behavior A is performed under a circumstance where the behavior A is not usually performed or the behavior A is not performed under a circumstance where the behavior A is usually performed, a sensing failure or false sensing occurs. A behavior habit may be simply referred to as a habit below.
In one aspect, an object is to provide a behavior sensing device, a behavior sensing method and a behavior sensing program that enable inhibition of occurrence of a failure to sense a behavior or false sensing of the behavior.

[Solution to Problem]

According to an aspect, a behavior sensing device includes: an acquisition unit configured to acquire sensor data from one or more sensors; a first characteristic amount calculator configured to calculate a first characteristic amount serving as an index of characteristic of biological reaction to a behavior to be sensed from the sensor data; a first probability calculator configured to calculate a probability of occurrence of the behavior as a first behavior occurrence probability from the first characteristic amount; a second characteristic amount calculator configured to calculate a second characteristic amount serving as an index of characteristic of a habit relating to the behavior to be sensed from the sensor data; a second probability calculator configured to calculate a probability of occurrence of the behavior as a second behavior occurrence probability from the second characteristic amount; and a determination unit configured to determine whether the behavior is performed based on the first behavior occurrence probability and the second behavior occurrence probability. The above described biological reactions are such as a signal representing a state and an activity of inside of a body and a body surface, and a signal capturing movement of a muscle and a region, for example, a blood pressure, body temperature, an electric skin resistance, an electrocardiographic waveform, a heart rate, a pulse waveform, a heart rate, a pulse rate, cutaneous temperature, myoelectric potential, phonation, an activity sound of an organ, a face image, and acceleration and an angular rate of an area of a body, or the like.

[Advantageous Effects of Invention]

It is possible to inhibit occurrence of a failure to sense a behavior and false sensing of a behavior.

[Brief Description of Drawings]

FIG. 1 is a diagram illustrating a configuration of a healthcare support system according to Exemplary Embodiment 1.
FIG. 2 is a diagram representing exemplary results of behavior sensing.
FIG. 3 is a diagram representing exemplary results of behavior sensing.
FIG. 4 is a diagram representing exemplary results of behavior sensing.
FIG. 5 is a diagram representing exemplary results of behavior sensing.
FIG. 6 is a diagram representing exemplary results of behavior sensing.
FIG. 7 is a flowchart representing a procedure of a learning process according to Exemplary Embodiment 1.
FIG. 8 is a flowchart representing a procedure of a behavior sensing process according to Exemplary Embodiment 1.
FIG. 9 is a diagram illustrating an exemplary hardware configuration of a computer that executes a behavior sensing program according to Exemplary Embodiment 1 and Exemplary Embodiment 2.

[Embodiments for Carrying Out the Invention]

A behavior sensing device, a behavior sensing method and a behavior sensing program according to the present application will be described below with reference to the accompanying drawings. Note that this exemplary embodiment does not limit the disclosed technology. Each exemplary embodiment can be combined as appropriate within the scope where the content of process is not inconsistent.

Exemplary Embodiment 1

System Configuration

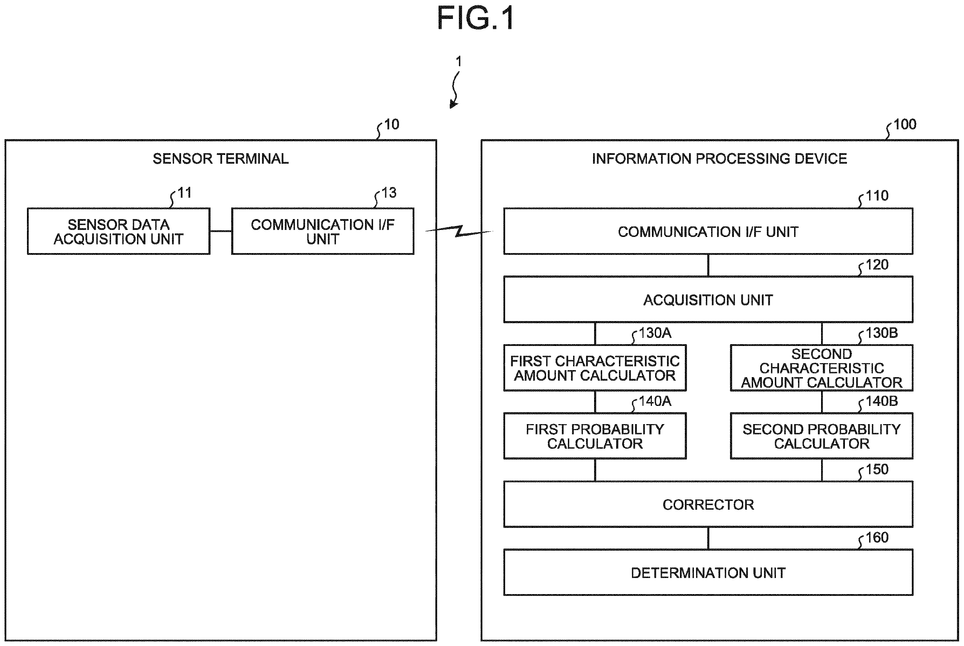

FIG. 1 is a diagram illustrating a configuration of a healthcare support system according to Exemplary Embodiment 1. A healthcare support system 1 illustrated in FIG. 1 provides health care support services that support health management for the lifestyle and health condition of a user of a sensor terminal 10, that is, health care, using sensor data that is collected by the sensor terminal 10. A case where a behavior, "eating", is to be sensed as a behavior of a user as part of the health care support services will be assumed and described below as an example only.
As illustrated in FIG. 1, the healthcare support system 1 includes the sensor terminal 10 and an information processing device 100. FIG. 1 illustrates the case where there is a single sensor terminal, and the healthcare support system 1 may house multiple sensor terminals.
The sensor terminal 10 and the information processing device 100 are connected such that they can communicate with each other. A case where the sensor terminal 10 and the information processing device 100 are connected to each other by near field communication, such as BLE (Bluetooth (trademark) low emergency), is assumed as an example herein, and the sensor terminal 10 and the information processing device 100 can be connected with each other via any network regardless whether the communication is wireless or wired. For example, the sensor terminal 10 and the information processing device 100 can be connected via any type of communication network including internal communication networks, such as a local area network (LAN) and a virtual private network (VPN), and the Internet.
The sensor terminal 10 is a terminal device that mounts a sensor.
In one embodiment, IoT devices including general-purpose wearable sensors, such as smart glasses and smart watches, and terminal devices dedicated to healthcare can be used for the sensor terminal 10.
At least one sensor is mounted on the sensor terminal 10. For example, in a case where eating performed by the user is to be sensed, sensors including a heart rate sensor, a motion sensor, such as an acceleration sensor or a gyro sensor, or a global positioning system (GPS) receiver can be mounted on the sensor terminal 10 as an example only.
A heart rate sensor from among the sensors will be taken as an example. When a heart rate sensor is mounted on the sensor terminal 10, a wearable heart rate sensor to be worn on a biological part of the user, such as the chest, an arm, or a wrist, is usable and a photoplethysmographic sensor to measure pulses is also usable. In this case, the heart rate sensor can be mounted only for health care or, when a heart rate sensor is mounted on a wearable gadget, that heart rate sensor can be used. A wearable heart rate sensor need not necessarily be used. For example, by detecting a heart rate from changes over time in the brightness of an image of a biological part of the user that is captured at a given sampling frequency or by detecting a Doppler frequency associated with heart beats with a radio frequency (RF) motion sensor, detection of a heart rate may be realized without any contact with any biological part of the user.
A "heart rate" herein is an index representing the number of heart beats of a heart that sends out blood and a method of sensing a heart rate may be a method of measuring cardiac electrical activities or a method of measuring a blood flow to measure pulsation. In other words, a heart rate sensor need not necessarily be mounted to detect a heart rate and an electrocardiogram sensor that detects electrocardiogram signals may be mounted on the sensor terminal 10.

The sensor data that is measured by the sensors mounted on the sensor terminal 10 is transmitted in association with identification information of the user, such as a machine name and a serial number of the sensor terminal 10, to the information processing device 100. The sensor data may be transmitted in realtime each time a sensor value is collected or may be stored for a given period, such as 12 hours, a day, a week, or a month, and then transmitted. The case where sensor data is transmitted from the sensor terminal 10 to the information processing device 100 is exemplified herein, and an amount of characteristic that is calculated from the sensor data, that is, a first characteristic amount and a second characteristic amount that are used to sense eating, which will be described below, may be transmitted from the sensor terminal 10 to the information processing device 100.

The information processing device 100 is a computer that provides the above-described healthcare support services. For the information processing device 100, computers including portable terminal devices and desktop and laptop personal computers are usable. The portable terminal devices include not only mobile communication terminals, such as smartphones, mobile phones and personal handyphone systems (PHS), but also tablet terminals and slate terminals.

In one embodiment, the information processing device 100 can be implemented by installing a behavior sensing program that realizes the above-described healthcare support services in an intended computer as package software or online software. For example, the information processing device 100 uses sensor data that is received from the sensor terminal 10 to sense eating performed by the user of the sensor terminal 10. The information processing device 100 can then record times of eating first, using the results of sensing eating, and furthermore generate a list of times of eating over a given period, such as a week, from the times of eating recorded before and output the list and perform analysis on dietary habits or dieting from the times of eating recorded before and then output various advices. For example, it is possible to output the above-described various types of information via an output device of the information processing device 100, such as a display device, a sound output device or a printing device. A destination to which the information is output is not necessarily limited to the information processing device 100, and the destination can be another terminal device that the user uses or a terminal device that a person who relates to the user, such as a relative of the user or a responsible person for medicine or nursing. Accordingly, the above-described healthcare support services are realized.

Configuration of Sensor Terminal 10

A functional configuration of the sensor terminal 10 according to the exemplary embodiment will be described. As illustrated in FIG. 1, the sensor terminal 10 includes a sensor data acquisition unit 11 and a communication I/F (interface) unit 13. The sensor terminal 10 may include functional units of a known computer in addition to the functional units represented in FIG. 1. For example, a wearable gadget, a terminal device dedicated to healthcare or a portable terminal device is implemented as the sensor terminal 10, hardware and software that each device includes as standard can be implement.

The sensor data acquisition unit 11 is a processor that controls sensors, which are not represented in the drawings, to acquire the above-described sensor data.

A case where the sensor data acquisition unit 11 acquires data of heart rates over time as exemplary sensor data will be exemplified as one aspect. For example, the sensor data acquisition unit 11 controls a heart rate sensor, which is not represented in the drawings, and causes the heart rate sensor to sense a heart rate in each given sampling period. Accordingly, the sensor data acquisition unit 11 acquires data of heart rates over time, which are sensed by the heart rate sensor at respective sampling points, as the heart rate data. For the heart rate data, for example, data in which items of time, heart rate, etc., are associated with each other can be used. The "time" herein may be a system time that is locally managed in the sensor terminal 10, such as a time elapsing from a given start point, or a time that is represented by the calendar on a calendar by year, month, hour, minute, second, etc. The "heart rate" is represented as a heart rate per unit of time. For example, when the unit of time is one minute, the heart rate is represented by beats per minute (bpm). When the unit of time is one second, the heart rate is represented by Hz. Furthermore, a heart rate need not be used when an index correlating with the "heart rate" is used. For example, an electrocardiographic RR interval in an electrocardiogram waveform is represented by milliseconds and may be used instead of a heart rate.

The purpose of acquisition of heart rates with the sensor terminal 10 is to capture responses of circulatory organs and use the responses to estimate a time of eating and, when an index, other than heart rates, correlating with the heart rates is obtained from information obtained from an electrocardiogram waveform and a pulse waveform and information relating to the amount of blood flow, the index may be used.

A case where the sensor data acquisition unit 11 acquires data of acceleration over time as exemplary sensor data will be exemplified as another aspect. For example, the sensor data acquisition unit 11 controls an acceleration sensor, which is not illustrated in the drawings, and causes the acceleration sensor to sense acceleration in each given sampling period in three axes, that is, vertically, horizontally and anteroposteriorly. Accordingly, the sensor data acquisition unit 11 acquires vertical, horizontal and anteroposterior data of acceleration over time, which are sensed by the acceleration sensor at respective sampling points, as acceleration data. For the acceleration data, for example, data in which items of time, acceleration, etc., are associated with each other can be used. The "time" herein may be, as in the case of the above-described heart rate data, a system time that is locally managed in the sensor terminal 10, such as a time elapsing from a given start point, or a time that is represented by the calendar on a calendar by year, month, hour, minute, second, etc. The "acceleration" can include vertical, horizontal and anteroposterial acceleration in three axes. For example, when acceleration in part of the three axes is emphasized and functional units at subsequent stages are caused to use the emphasized acceleration, the acceleration in a direction not to be used by the functional unit at subsequent stages can be removed from the acceleration data.

As described above, it is also possible to acquire sensor data other than heart rate data and acceleration data. For example, it is also possible to acquire location data in which each time is associated with a location measured by a GPS receiver at the time, such as the latitude and longitude.

The communication I/F unit 13 is an interface that performs communication control with another device, for example, the information processing device 100.

In one embodiment, a BLE module is usable for the communication I/F unit 13 when the sensor terminal 10 and the information processing device 100 are connected with each other by near field communication. Alternatively, when they are connected with each other by a wireless communication network, such as a LAN or a VLAN, a network interface card, such as a LAN card, is usable for the communication I/F unit 13. For example, the communication I/F unit 13 transmits the above-described sensor data, etc., to the information processing device 100. Furthermore, the communication I/F unit 13 can receive, in addition to an instruction to upload the above-described sensor data, etc., to the information processing device 100 and an instruction about intervals at which the above-described sensor data is uploaded to the information processing device 100, support information, such as results of sensing behaviors and diagnosis results obtained by using the sensing results, from the information processing device 100.

As described above, the sensor data that is detected by the sensor data acquisition unit 11 is transmitted by the communication I/F unit 13 to the information processing device 100 according to an instruction of a controller, such as a central processing unit (CPU) or a micro-processing unit (MPU), which are not represented in the drawings. Each time a sensor value is sampled, the sensor value may be transmitted to the information processing device 100, or sensor data may be stored in a memory, which is not represented in the drawings, over a given period, such as 12 hours, a day, a week or a month, and then transmitted to the information processing device 100.

For a main storage device that is used by a processor, such as the sensor data acquisition unit 11, for example, various semiconductor memory devices, such as a random access memory (RAM) or a flash memory, are usable. The storage device to which each of the above-described processor refers is not necessarily the main storage device and an auxiliary storage device may be referred to. In that case, a hard disk drive (HDD), an optical disk or a solid state drive (SSD) is usable.

Configuration of Information Processing Device 100

A functional configuration of the information processing device 100 according to the exemplary embodiment will be described. As illustrated in FIG. 1, the information processing device 100 includes a communication I/F unit 110, an acquisition unit 120, a first characteristic amount calculator 130A, a second characteristic amount calculator 130B, a first probability calculator 140A, a second probability calculator 140B, a corrector 150 and a determination unit 160. The information processing device 100 may include, in addition to the functional units represented in FIG. 1, functional units of a known computer, such as various input/output devices.

The communication I/F unit 110 is an interface that controls communication with other devices, such as the sensor terminal 10.

In one embodiment, when the sensor terminal 10 and the information processing device 100 are connected to each other by near field communication, a BLE module or the like is usable for the communication I/F unit 110. When the connection is implemented by a wireless communication network, such as a LAN or a VLAN, a network interface card, such as a LAN card, is usable for the communication I/F unit 110. For example, the communication I/F unit 110 receives the above-described sensor data, etc., from the sensor terminal 10. Furthermore, the communication I/F unit 110 is able to transmit, in addition to an instruction for the sensor terminal 10 to upload the above-described sensor data, etc., and an instruction about an interval at which the sensor terminal 10 uploads the sensor data to the information processing device 100, support information, such as results of sensing behaviors and diagnosis results obtained by using the sensing results, to the sensor terminal 10.

The acquisition unit 120 is a processor that acquires the above-described sensor data.

In one embodiment, the acquisition unit 120 is able to acquire the sensor data via near field wireless communication from the sensor terminal 10. The acquisition unit 120 is also able to acquire heart rate data by, in addition to access by such communication, reading the sensor data saved in an auxiliary device, such as a hard disk or an optical disk, or in a removable medium, such as a memory card or a universal serial bus (USB) memory. The case where the sensor data is acquired from the sensor terminal 10 by near field wireless communication is exemplified herein. In a case where the sensor terminal 10 incorporates or provided with the sensors, which are described with respect to the sensor terminal 1, the information processing device 100 may directly acquire sensor data that is output by the sensors.

A processor in a subsequent stage to which the sensor data is input from the acquisition unit 120 represented in FIG. 1 bifurcates into two processing systems in which processes are executed in parallel. In other words, the processor is roughly divided into a first processing system that performs a process relating to the first characteristic amount serving as an index of characteristic of biological reaction to a behavior and a second processing system that performs a process relating to the second characteristic amount serving as an index of characteristic of habit relating to the behavior. For example, the first processing system includes the first characteristic amount calculator 130A and the first probability calculator 140A. The second processing system includes the second characteristic amount calculator 130B and the second probability calculator 140B. Each of the processors included by the first processing system will be described and then each of the processors included by the second processing system will be described below.

The first characteristic amount calculator 130A is a processor that calculates the above-described first characteristic amount. A case where a change in the heart rate is calculated as an exemplary first characteristic amount is exemplified as an example only herein and, needless to say, an existing index can be calculated alternatively as the first characteristic amount as long as the index represents a characteristic of a biological reaction to eating and calculation of multiple first characteristic amounts is not inhibited.

In one embodiment, the first characteristic amount calculator 130A chooses sensor data to be used to calculate the first characteristic amount to be calculated from among the sensor data that is acquired by the acquisition unit 120. For example, when the above-described "a change in the heart rate" is calculated as the first characteristic amount, a setting to input the heart rate data from among the sensor data acquired by the acquisition unit 120 is made in a work area of an internal memory, which is not represented in the drawings. The first characteristic amount calculator 130A refers to the setting and thus extracts the heart rate data from among the sensor data acquired by the acquisition unit 120 and calculates a change in the heart rate. For example, the first characteristic amount calculator 130A calculates, for each time contained in the heart rate data, a difference between the heart rate that is measured at the time and the heart rate that is measured right before the time as a change in the heart rate. Accordingly, the first characteristic amount is obtained for each of the times. The case where a difference in the heart rate between a current time and a time right before the current time is calculated as a change in the heart rate has been exemplified herein, and a difference between the heart rate at the current time and a statistical value of heart rates measured before a given period back from the current time, for example, an average, can be calculated as a change in the heart rate or a difference between the heart rate at the current time and a statistical value of heart rates over the whole period can be calculated as a change in the heart rate.

The first probability calculator 140A is a processor that calculates, as a first behavior occurrence probability, a probability of occurrence of a behavior with which the behavior occurs from the first characteristic amount.

In one embodiment, the first probability calculator 140A calculates, for each of the times, a first behavior occurrence probability from a first determination model to classify the first characteristic amount into eating or not-eating class and the first characteristic amount that is calculated for each of the times by the first characteristic amount calculator 130A. For example, the first behavior occurrence probability can be calculated according to an algorithm, such as logistic regression. The "first determination model" is data that is generated by performing machine learning using first learning data in which the eating or not-eating label and the first characteristic amount are associated with each other for each of the times and that is, after being saved in the work area of the internal memory, or the like, in the stage of machine learning, referred to in a stage to perform behavior sensing. The machine learning need not necessarily be executed by the information processing device 100, and the first determination model that is generated by another device may be saved in the work area in the internal memory.

The second characteristic amount calculator 130B is a processor that calculates the above-described second characteristic amount. A case where "presence or absence of exercise" and "the category of the current location" are calculated as an exemplary second characteristic amount is exemplified as an example only herein and, needless to say, an existing index can be calculated alternatively as the second characteristic amount as long as the index represents a characteristic of a habit relating to the behavior and calculation of multiple second characteristic amounts is not inhibited.

In one embodiment, the second characteristic amount calculator 130B chooses sensor data to be used to calculate the second characteristic amount to be calculated from among the sensor data that is acquired by the acquisition unit 120. For example, when the above-described "presence or absence of exercise" and "the category of the current location" are calculated as the second characteristic amount, a setting to input the acceleration data from among the sensor data acquired by the acquisition unit 120 is made in the work area of the internal memory, or the like. The second characteristic amount calculator 130B refers to the setting and thus extracts the acceleration data and the location data from among the sensor data acquired by the acquisition unit 120 and calculates presence or absence of exercise. For example, the second characteristic amount calculator 130B calculates, for each time contained in the acceleration data, presence or absence of walking, ascending and descending, or running as presence or absence of exercise from the acceleration that is measured at the time. The second characteristic amount calculator 130B calculates, for each time contained in the location data, the result of classification of the location that is measured at the time into a category of home, workplace, railway or restaurant as the category of the current location. Accordingly, the second characteristic amount is obtained for each of the times.

The second probability calculator 140B is a processor that calculates, as a second behavior occurrence probability, a probability of occurrence of a behavior with which the behavior occurs from the second characteristic amount.

In one embodiment, the second probability calculator 140B calculates, for each of the times, a second behavior occurrence probability from a second determination model to classify the second characteristic amount into eating or not-eating class and the second characteristic amount that is calculated for each of the times by the second characteristic amount calculator 130B. For example, the second behavior occurrence probability can be calculated according to an algorithm, such as logistic regression. The "second determination model" is data that is generated by performing machine learning using second learning data in which the eating or not-eating label and the second characteristic amount are associated with each other for each of the times and that is, after being saved in the work area of the internal memory in the stage of machine learning, referred to in a stage to perform behavior sensing. The machine learning need not necessarily be executed by the information processing device 100, and the first determination model that is generated by another device may be saved in the work area in the internal memory.

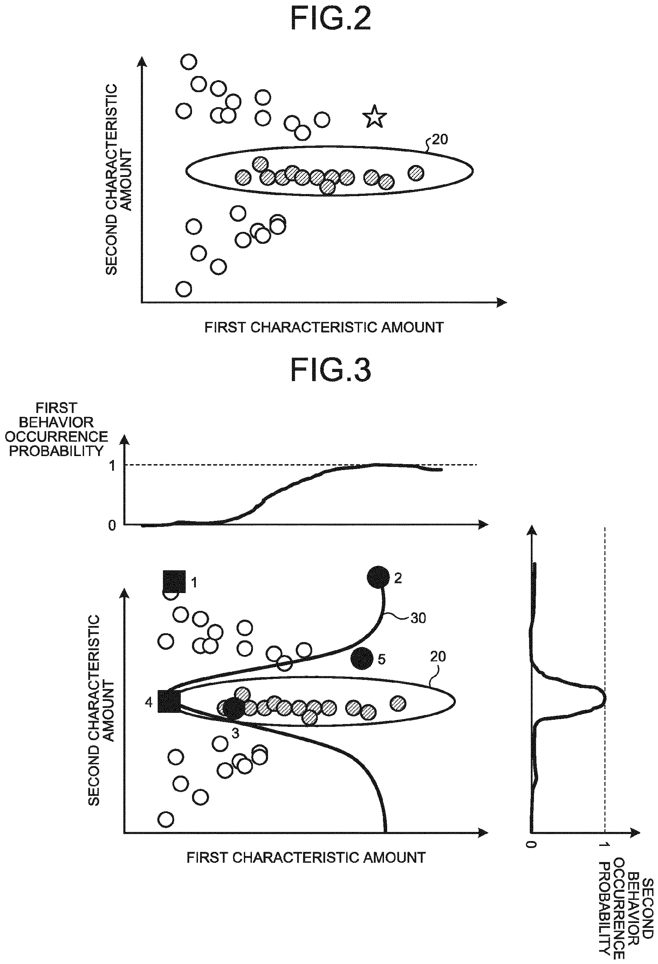

FIG. 2 is a diagram representing exemplary results of behavior sensing. FIG. 2 represents results of performing behavior sensing according to the determination model obtained by performing machine learning using both the first characteristic amount and the second characteristic amount as characteristic vectors. FIG. 2 further represents a case where a change in the heart rate is calculated as the first characteristic amount and a time is calculated as the second characteristic amount and, while characteristic vectors classified into eating are represented as hatched, characteristic vectors classified into not-eating are represented as filled with white. The reference numeral 20 represented in FIG. 2 denotes a boundary by which the characteristic vectors are classified into eating or not-eating according to the determination model. The horizontal axis of the graph represented in FIG. 2 represents a change in the heart rate and the horizontal axis represents a time.

For example, in a case of a user who takes a meal at almost the same time every day, a boundary between a positive example (eating) and a negative example (not-eating), that is, a boundary by which characteristic vectors are classified into eating or not-eating according to the determination model is as denoted by the reference numeral 20 represented in FIG. 2. For this reason, in a case where a meal is taken earlier one day, for example, when a characteristic vector of the star-shaped plot in FIG. 2 is calculated, although a change in the heart rate represents a possibility of eating, an amount of characteristic of habit is different from a normal one and thus it is not possible to sense the behavior as eating. As described above, the following is taken as a reason for the boundary, denoted by the reference numeral 20, by which classification into eating or not-eating is performed according to the determination model. Specifically, as for a biological reaction, while occurrence of a signal change associated with a behavior to be sensed is highly reliable, the signal change to occur tends to vary widely. On the other hand, as for the habit relating to the behavior, while whether a signal change associated with the behavior to be sensed occurs is stochastic, the signal change to occur tends to vary small. As for habits, as people tend to repeat the same habitual pattern every day, occurrence of a signal change tends to seem to be highly reliable in the data. Machine learning, etc., prioritizes an amount of characteristic for which a signal change to occur highly reliably varies small and thus such an event occurs.

By separately dealing with a biological reaction for which occurrence of a signal change associated with the behavior to be sensed is highly reliable and the habit for which whether a signal change associated with a behavior to be sensed relates to a stochastic behavior, it is possible to, even when a behavior against the habit is performed, determine whether the behavior is performed. In the embodiment, the sensor data is separated into a characteristic of a biological reaction to a behavior and a characteristic of the habit that relates to the behavior to calculate the first characteristic and the second characteristic and individual behavior occurrence probabilities thereof are used for behavior sensing. For example, when the sum of a first behavior occurrence probability and a second behavior occurrence probability is a threshold or larger, for example, 1 or larger, it can be determined that the behavior is performed.

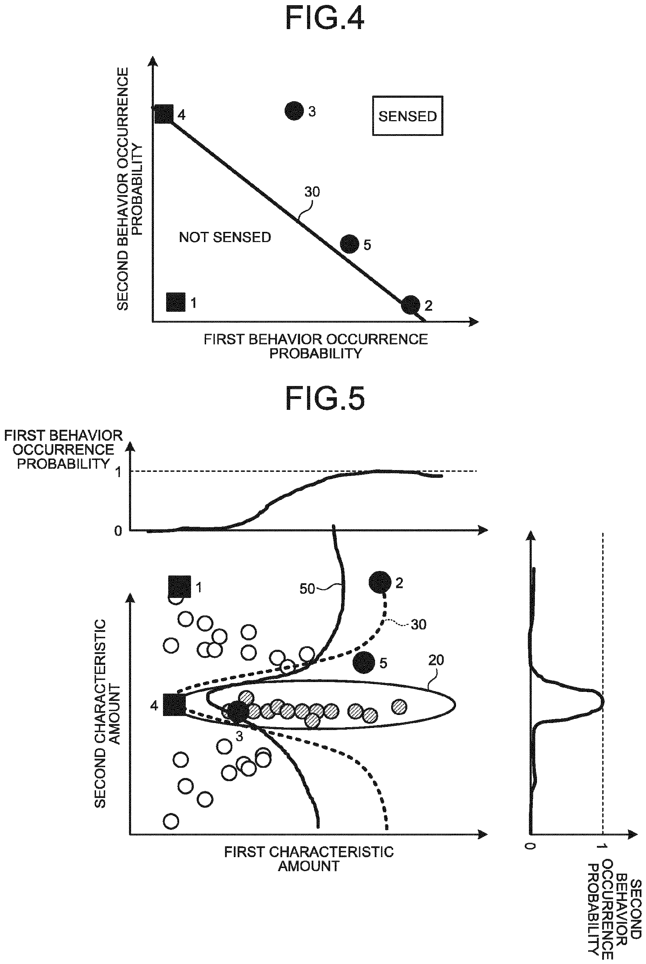

FIGS. 3 and 4 are diagrams representing exemplary results of behavior sensing. FIGS. 3 and 4 represent results of behavior sensing performed according to whether the sum of the first behavior occurrence probability and the second behavior occurrence probability is one or larger. As FIG. 2 represents, FIG. 3 represents a case where a change in the heart rate is calculated as the first characteristic amount and a time is calculated as the second characteristic amount. Furthermore, in FIG. 3, while plots classified into eating among sets of first characteristic amounts and second characteristic amounts that are calculated at the same time are represented as hatched, plots classified into not-eating are represented as filled with white. Furthermore, in FIG. 3, two graphs representing changes in the first behavior occurrence probability and the second behavior occurrence probability above and on the right of the graph containing the first characteristic amount and the second characteristic amount in the horizontal axis and the vertical axis are represented according to the horizontal axis or the vertical axis of the previous graph. The horizontal axis of the graph represented in FIG. 3 indicates a change in the heart rate and the vertical axis indicates a time. The horizontal axis of the graph represented in FIG. 4 indicates a first behavior occurrence probability and the horizontal axis indicates a second behavior occurrence probability.

For example, a boundary by which the eating or not-eating is determined using the first behavior occurrence probability and the second behavior occurrence probability is as denoted by the reference numeral 30 represented in FIGS. 3 and 4. The plots 1 to 5 represented as filled with black will be focused on. From among the plots 1 to 5, the plots 2, 3 and 5 for which the actual behaviors are eating are represented in a circular shape and the plots 1 and 4 for which the actual behaviors are not-eating are represented in a square shape. As indicated by the boundary 30 represented in FIGS. 3 and 4, it is represented that, when eating or not-eating is determined from the first behavior occurrence probability and the second behavior occurrence probability, the plot 5 that is detected as not-eating as a result of detection failure according to the boundary 20 of the determination model represented in FIG. 2 can be determined as "eating".

As described above, as a result of performing behavior sensing using the first behavior occurrence probability and the second behavior occurrence probability equally, it is possible to inhibit behavior sensing from being performed according to determination weighting the characteristic of the habit relating to the behavior due to machine learning. In other words, it is possible to inhibit occurrence of a failure of sensing and false sensing in a case where a behavior against the habit is performed, such as a case where eating is performed under a circumstance where eating is not performed usually or a case where eating is not performed under a circumstance where eating is performed usually. For example, in the examples according to FIGS. 3 and 4, the plot 5 of eating at a time when eating tends not to be performed usually can be sensed as eating.

The corrector 150 is a processor that corrects priority of the first behavior occurrence probability and the second behavior occurrence probability.

The corrector 150 corrects the priority according to three principles. A first principle is that, when the first behavior occurrence probability is high, priority of the second behavior occurrence probability is lowered. The principle is taken because, when a biological reaction is detected clearly from the heart rate data, etc., it is better to determine that eating is performed even when the detection is performed from acceleration, a location and a time at and in which a meal is not taken usually. A second principle is that, when the first behavior occurrence probability is intermediate or low, priority of the second behavior occurrence probability is increased. The principle is taken because, when it is difficult to detect a biological reaction clearly from the heart rate data, etc., the determination is made in consideration of the habit regarding whether it is time to take a meal usually. A third principle is that, when the first behavior occurrence probability is low significantly, priority of the second behavior occurrence probability is lowered. The principle is taken because, when a biological reaction is hardly detected from the heart rate data, etc., it is better not to determine that eating is performed just because acceleration, a location and a time similar to those of the habit of eating are detected.

In order to implement the above-described principles (1) and (3), the corrector 150 changes the weight of the second behavior occurrence probability, which is calculated by the second probability calculator 140B, to a small one regardless of the value of the first behavior occurrence probability, which is calculated by the first probability calculator 140A. For example, the corrector 150 lowers the weight of the second behavior occurrence probability, which is calculated by the second probability calculator 140B, according to Expression (1) given below. "a" in Expression (1) given below is a weight of the second behavior occurrence probability and satisfies the condition $0<a<1$ and, for example, the smallest value from among experimental values by which positive examples and negative examples can be distinguished most clearly in the first learning data and the second learning data that are used to learn the first determination model and the second determination model is used for "a".

$$\text{First behavior occurrence probability} + a * \text{Second behavior occurrence probability} > (1+a)/2 \qquad (1)$$

When implementing the above-described principles (1) to (3), the corrector 150 corrects the first behavior occurrence probability that is calculated by the first probability calculator 140A according to Expression (2) given below as an example only. "x" in Expression (2) denotes a value of the first behavior occurrence probability that is calculated by the first probability calculator 140A. By assigning the value to Expression (2), it is possible to calculate the first behavior occurrence probability after the correction. In other words, when the first behavior occurrence probability takes a value around 1 and 0, it is possible to adjust the probability using a function by which the obliqueness is larger than that with 1. Expression (2) below is an example and, needless to say, it is possible to use another geometric expression using arccos, or the like, as long as the geometric expression implements the principles (1) to (3). The case where the first behavior occurrence probability, which is calculated by the first probability calculator 140A, is corrected is exemplified herein, and the second behavior occurrence probability, which is calculated by the second probability calculator 140B, may be corrected.

$$\tan((x*2-1)*\pi/2-\pi/2) \qquad (2)$$

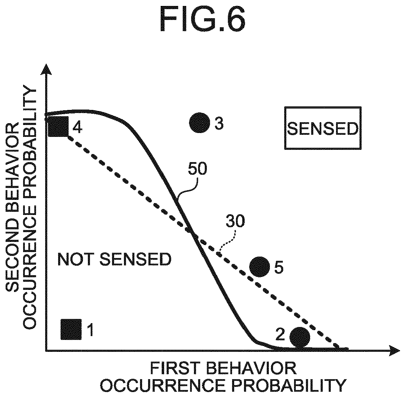

FIGS. 5 and 6 are diagrams representing exemplary results of behavior sensing. FIGS. 5 and 6 represent results of performing behavior sensing according to a third determination model obtained by performing machine learning from the first behavior occurrence probability that is corrected according to Expression (2) given above and the second behavior occurrence probability. Furthermore, as FIG. 2 represents, FIG. 5 represents the case where a change in the heart rate is calculated as the first characteristic amount and a time is calculated as the second characteristic amount. Furthermore, in FIG. 5, while plots classified into eating among sets of first characteristic amounts and second characteristic amounts that are calculated at the same time are represented as hatched, plots classified into not-eating are represented as filled with white. Furthermore, in FIG. 5, two graphs representing changes in the first behavior occurrence probability and the second behavior occurrence probability above and on the right of a graph containing the first characteristic amount and the second characteristic amount in the horizontal axis and the vertical axis are represented according to the horizontal axis or the vertical axis of the previous graph. The horizontal axis of the graph represented in FIG. 5 indicates a change in the heart rate and the vertical axis indicates a time. The horizontal axis of the graph represented in FIG. 6 indicates a first behavior occurrence probability and the horizontal axis indicates a second behavior occurrence probability.

For example, a boundary by which eating or not-eating is determined according to the third determination model is as denoted by the reference numeral 50 represented in FIGS. 5 and 6. In the case of the boundary 50 represented in FIGS. 5 and 6, the plot 4 that is falsely sensed as eating according to the boundary 30 represented in FIGS. 3 and 4 can be determined as "not-eating" and the plot 2 that is detected as not-eating as a result of detection failure according to the boundary 30 represented in FIGS. 3 and 4 can be determined as "eating".

As a result of performing behavior sensing according to the third determination model that is obtained by performing machine learning from the first behavior occurrence probability that is corrected according to Expression (2) given above and the second behavior occurrence probability, it is possible to inhibit behavior sensing from being performed by determination weighting the characteristic of the habit relating to the behavior due to machine learning. For example, in the examples according to FIGS. 5 and 6, the plot 2 of eating at a time when eating tends not to be performed usually can be sensed as eating and he plot 4 of not-eating at a time when eating is performed usually can be sensed as not-eating.

The determination unit 160 is a processor that determines whether a behavior is performed from the first behavior occurrence probability and the second behavior occurrence probability.

In one embodiment, for example, as the first behavior occurrence probability that is corrected according to Expression (2) given above and the second behavior occurrence probability are classified into the eating or not-eating class, the determination unit 160 calculates, for each of the times, whether it is eating or not-eating from the first behavior occurrence probability, which is corrected for each of the times by the corrector 150, and the second behavior occurrence probability, which is calculated by the second probability calculator 140B, using the third determination model. The "third determination model" is data that is generated by performing machine learning using third learning data in which the eating or not-eating label, the corrected first behavior occurrence probability, and the second behavior occurrence probability are associated with one another for each of the times and that is, after being saved in the work area of the internal memory, or the like, in the stage of machine learning, referred to in a stage to perform behavior sensing. The machine learning need not necessarily be executed by the information processing device 100, and the first determination model that is generated by another device may be saved in the work area in the internal memory.

The times of eating that are estimated using the results of estimation of behavior performed by the determination unit 160, for example, any one of a set of times at which eating starts, a set of times at which eating ends and a set of times needed for eating, is output to a unit that executes the healthcare support services to record times of eating, generate a list of times of eating over a given period, such as a week, and then output the list, and analyze the dietary habits or dieting from the times of eating that are recorded before and then output various advices. The unit that executes the healthcare support services may be a processor that is implemented in the processor of the information processing device 100 or may be an external server device, or the like.

The processors including the acquisition unit 120, the first characteristic amount calculator 130A, the second characteristic amount calculator 130B, the first probability calculator 140A, the second probability calculator 140B, the corrector 150 and the determination unit 160 can be implemented in the following manner. For example, the processors can be implemented by causing a central processing device, such as a CPU, to load processes to implement the same functions as the acquisition unit 120, the first characteristic amount calculator 130A, the second characteristic amount calculator 130B, the first probability calculator 140A, the second probability calculator 140B, the corrector 150 and the determination unit 160 in a memory and execute the processes. The processors need not necessarily be implemented by the central processing device and may be implemented by a MPU. Each of the above-described functional units can be implemented by using a hardwired logic.

For example, various semiconductor memory devices, such as a RAM and a flash memory, can be used for the main storage device used by each of the above-described processors. The storage device that is referred to by each functional unit need not necessarily be the main storage device and it may be an auxiliary storage device. In that case, a HDD, an optical disk or a SSD is usable.

Flow of Processes

A flow of the processes of the information processing device 100 according to the exemplary embodiment will be described. After (1) Learning Process that is executed by the information processing device 100 is described, (2) Behavior Sensing Process will be described.

(1) Learning Process

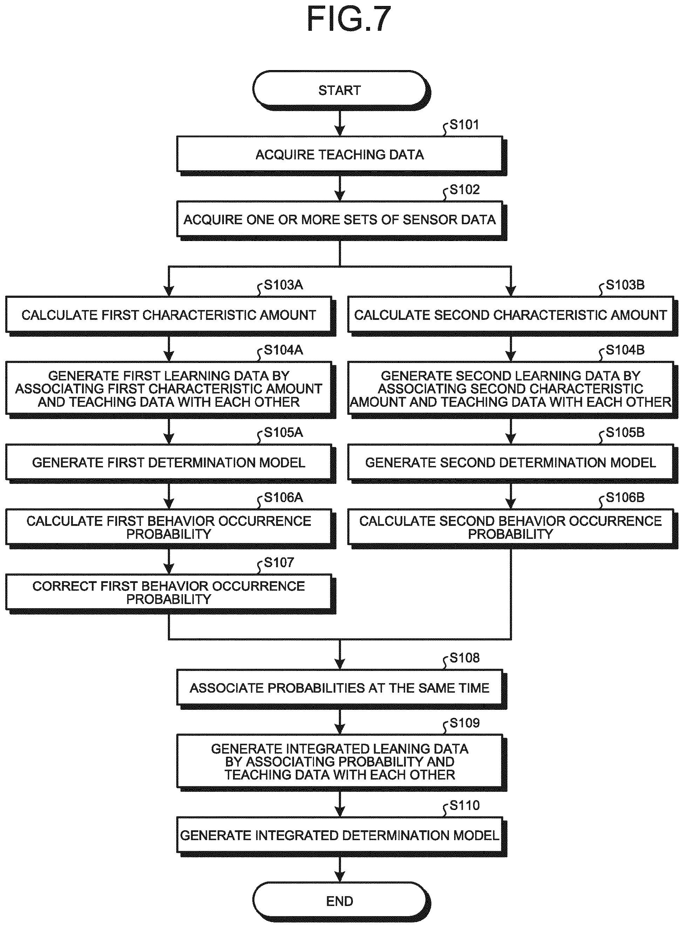

FIG. 7 is a flowchart representing a procedure of the learning process according to Exemplary Embodiment 1. The learning process, for example, can be started at any timing at which a destination to which teaching data is supplied is confirmed. As represented in FIG. 7, teaching data is acquired from a given destination of supply (step S101) and the acquisition unit 120 acquires one or more sets of sensor data (step S102). For the teaching data, for example, it is possible to use data with which a period during which eating is actually performed can be specified, such as data that is a time at which eating starts and a time at which eating ends and that is input by a user, or the like.

Process A including the subsequent steps S103A to S106A and step S107 and Process B including steps S103B to S106B are executed in parallel.

First of all, Process A including steps S103A to S106A and step S107 will be described. After execution of step S102, the first characteristic amount calculator 130A calculates, for each of times contained in the sensor data acquired at step S102, a first characteristic amount from a sensor value that is measured at the time (step S103A).

The first characteristic amount calculator 130A adds an eating or not-eating label according to, for each of the times for which the first characteristic amount is calculated at step S103A, whether the time is contained in the time at which eating starts and the time at which eating ends in the teaching data, which is acquired at step S101, to generate first learning data (step S104A). Accordingly, positive data to which the label "eating" is added and negative data to which the label "not-eating" is added can be prepared as first learning data.

Thereafter, the first probability calculator 140A generates a first determination model by performing machine learning using the first learning data, which is generated at step S104A (step S105A). Thereafter, the first probability calculator 140A calculates, for each of the times, a first behavior occurrence probability from the first determination model that is calculated at step S105A and the first characteristic amount that is calculated for each of the times at step S103A (step S106A). Subsequently, as an example only, the corrector 150 assigns the first behavior occurrence probability that is calculated at step S106A to "x" represented in Expression (2) given above to correct the first behavior occurrence probability (step S107).

Process B including steps S103B to S106B will be described. After execution of step S102, the second characteristic amount calculator 130B calculates, for each of the times contained in the sensor data acquired at step S102, a second characteristic amount from a sensor value that is measured at the time (step S103B).

The second characteristic amount calculator 130B adds an eating or not-eating label according to, for each of the times for which the second characteristic amount is calculated at step S103B, whether the time is contained in the time at which eating starts and the time at which eating ends in the teaching data, which is acquired at step S101, to generate second learning data (step S104B). Accordingly, positive data to which the label "eating" is added and negative data to which the label "not-eating" is added can be prepared as second learning data.

Thereafter, the second probability calculator 140B generates a second determination model by performing machine learning using the second learning data, which is generated at step S104B (step S105B). Thereafter, the second probability calculator 140B calculates, for each of the times, a second behavior occurrence probability from the second determination model that is calculated at step S105B and the second characteristic amount that is calculated for each of the times at step S103B (step S106B).

As described above, when Process A proceeds to step S107 and Process B proceeds to step S106B, the determination unit 160 associates the first behavior occurrence probability corrected at step S107 and the second behavior occurrence probability that is calculated at step S106B, which are the behavior occurrence probabilities tagged with the same time (step S108).

The determination unit 160 then adds the eating or not-eating label according to, for each of the times with which a pair of the corrected first behavior occurrence probability and the second behavior occurrence probability are associated at step S108, whether the time is contained in the time at which eating starts and the time at which eating ends in the teaching data, which is acquired at step S101, to generate third leaning data (step S109). Accordingly, positive data to which the label "eating" is assigned and negative data to which the label "not-eating" is added can be prepared as third learning data.

The determination unit 160 then generates a third determination model by performing machine leaning using the third leaning data, which is generated at step S109 (step S110), and ends the process.

Behavior Sensing Process

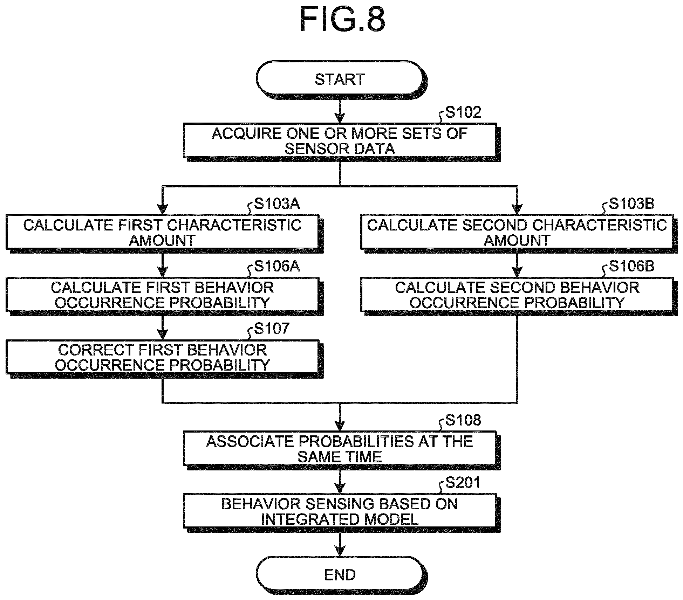

FIG. 8 is a flowchart representing a procedure of the behavior sensing process according to Exemplary Embodiment 1. The behavior sensing process, for example, can be started at any timing after the first model, the second model and the third model are generated. Note that processes whose content is common with that of the steps represented in FIG. 7, from among the steps represented in FIG. 8, are denoted with the same step numbers.

As illustrated in FIG. 8, when the acquisition unit 120 acquires one or more sets of sensor data (step S102), Process A at and after step S103A and Process B at and after step S103B are executed in parallel.

First of all, Process A will be described. After execution of step S102, the first characteristic amount calculator 130A calculates, for each of the times contained in the sensor data that is acquired at step S102, a first characteristic amount from a sensor value that is measured at the time (step S103A).

The first probability calculator 140A calculates, for each of the times, a first behavior occurrence probability from the first determination model that is generated by the leaning process represented in FIG. 7 and the first characteristic amount that is calculated for each of the times at step S103A (step S106A). Subsequently, as an example only, the corrector 150 assigns the first behavior occurrence probability that is calculated at step S106A to "x" represented in the Expression (2) given above to correct the first behavior occurrence probability (step S107) .

Process B will be described. After execution of step S102, the second characteristic amount calculator 130B calculates, for each of the times contained in the sensor data acquired at step S102, a second characteristic amount from the sensor value measured at the time (step S103B).

The second probability calculator 140B calculates, for each of the times, a second behavior occurrence probability from the second determination model that is generated by the leaning process represented in FIG. 7 and the second characteristic amount that is calculated for each of the times at step S103B (step S106B).

As described above, when Process A proceeds to step S107 and Process B proceeds to step S106B, the determination unit 160 associates the first behavior occurrence probability that is corrected at step S107 and the second behavior occurrence probability that is calculated at step S106B, which are the behavior occurrence probabilities tagged with the same time (step S108).

The determination unit 160 then classifies the first characteristic amount that is calculated at step S103A and the second characteristic amount that is calculated at step S103B into eating or not-eating according to the third determination model, which is generated by the learning process represented in FIG. 7, to sense whether eating is performed at each of the times (step S201) and ends the process.

One Aspect of Effect

As described above, the information processing device 100 according to the embodiment performs behavior sensing using behavior occurrence probabilities that are calculated individually for a characteristic amount representing a biological reaction to a behavior and a characteristic amount representing a habit of the behavior from sensor data. Accordingly, it is possible to inhibit performance of behavior sensing using determination emphasizing the characteristic of the habit relating to the behavior due to machine learning. Accordingly, according to the information processing device 100 according to the exemplary embodiment, it is possible to inhibit occurrence of a failure of sensing and false sensing when a behavior against a habit is performed.

Exemplary Embodiment 2

The exemplary embodiment relating to the disclosed device has been described; however, the present invention may be performed in various different modes in addition to the above-described exemplary embodiment. Other exemplary embodiments included by the present invention will be described.

Eating Sensing

Exemplary Embodiment 1 described above exemplifies the case where a change in the heart rate is calculated as the first characteristic amount, and an amount of another type of characteristic can be calculated as the first characteristic amount. For example, the information processing device 100 can calculate, as the first characteristic amount, presence or absence of motion of the throat, presence or absence of motion of arms, or expansion or shrinkage of the chest from a sensor value of an acceleration sensor or a gyro sensor. Exemplary Embodiment 1 described above exemplifies the case where presence or absence of exercise or a category of current location is calculated as the second characteristic amount, and an amount of another type of characteristic may be calculated as the second characteristic amount. For example, the information processing device 100 can calculate the ON or OFF operational status as a second characteristic amount from a camera.

Other Behavior Sensing

Exemplary Embodiment 1 exemplifies the case where eating sensing is performed as exemplary behavior sensing, and the information processing device 100 is usable for a behavior when a change in the biological reaction associated with the behavior is observable. The information processing device 100 is usable also to sense other behaviors, such as urination, egestion, water-drinking, alcohol-drinking, sitting down, standing up, walking, ascending and descending and sleeping.

(1) Urination

For example, when urination sensing is performed, an amount of the following characteristic can be calculated as the first characteristic amount. For example, a change in the heart rate can be calculated as the first characteristic amount from heart rate data that is acquired from a heart rate sensor. In another example, a rate of weight loss can be calculated as the first characteristic amount from weight data that is acquired from a load sensor. A rate of weight loss is defined as the first characteristic amount as described above because there is a characteristic that the weight lowers after urination.

When urination sensing is performed, an amount of the following characteristic can be calculated as the second characteristic amount. For example, presence or absence of walking, presence or absence of motion of the hands, and presence or absence of veering can be calculated as the second characteristic amount from motion data that is acquired from a motion sensor. They are defined as the second characteristic amount because there is a characteristic that walking is performed in order to go to a toilet before and after urination, a characteristic that the hands are often washed after urination, and a characteristic that veering is often performed in order to sit on a toilet seat. In another example, presence or absence of sound corresponding to the flow of water can be calculated as the second characteristic amount from sound data that is acquired from a microphone. Presence or absence of sound corresponding to the flow of water is defined as the second characteristic amount as described above because there is a characteristic that cleaning water often flows according to cleaning of a toilet bowl before and after urination.

(2) Egestion

When egestion sensing is performed, an amount of the following characteristic can be calculated as the first characteristic amount. For example, a change in the heart rate can be calculated as the first characteristic amount from heart rate data that is acquired from a heart rate sensor. In another example, a rate of weight loss can be calculated as the first characteristic amount from weight data that is acquired from a load sensor. A rate of weight loss is defined as the first characteristic amount as described above because there is a characteristic that the weight lowers after egestion.

When egestion sensing is performed, an amount of the following characteristic can be calculated as a second characteristic amount. For example, presence or absence of walking, presence or absence of motion of hands, and a posture of standing up or sitting down can be calculated as the second characteristic amount from motion data that is acquired from a motion sensor. They are defined as the second characteristic amount because there is a characteristic that walking is performed in order to go to a toilet before and after egestion, a characteristic that the hands are often washed after egestion, and a characteristic that veering is often performed in order to sit on a toilet seat before and after egestion.

(3) Water-drinking

When water-drinking sensing is performed, an amount of the following characteristic can be calculated as the first characteristic amount. For example, an increase and a decrease in the heart rate can be calculated as a first characteristic amount from heart rate data that is acquired from a heart rate sensor. They are defined as the first characteristic amount because there is a characteristic that the heart rate lowers after representing an abrupt increase in water drinking. In another example, a change in the body temperature can be calculated as the first characteristic amount from body temperature data that is acquired from a thermometer. A change in the body temperature is defined as the first characteristic amount as described above because there is a characteristic that the body temperature fluctuates due to the temperature of drink after water drinking.

When water-drinking sensing is performed, an amount of the following characteristic can be calculated as the second characteristic amount. For example, information, such as an increase in the temperature and information of the humidity, can be calculated as the second characteristic amount from temperature data and temperature data that are acquired from a temperature-temperature sensor. They are defined as the second characteristic amount because there is a characteristic that water drinking is often performed with an increase in the temperature and humidity. In another example, presence or absence of exercise can be calculated as the second characteristic amount from motion data that is acquired from a motion sensor. Presence or absence of exercise is defined as the second characteristic amount because there is a characteristic that water drinking is often performed after exercise.

(4) Alcohol-drinking

When alcohol-drinking sensing is performed, an amount of the following characteristic can be calculated as the first characteristic amount. For example, a change of increase in the heart rate can be calculated as the first characteristic amount from heart rate data that is acquired from a heart rate sensor. A change of increase in the heart rate is defined as the first characteristic amount as described above because there is a characteristic that the heart rate increases in alcohol drinking. In another example, similarity between the color of a face area and red can be calculated as the first characteristic amount from image data and spectral data that are acquired from an image sensor or the like. This is defined as the first characteristic because there is a characteristic that the face turns red after alcohol drinking.
When alcohol-drinking sensing is performed, an amount of the following characteristic can be calculated as the second characteristic amount. For example, time information can be calculated as the second characteristic information from time data that is acquired from a timer. It is defined as the second characteristic amount because there is a characteristic that the time of drinking is roughly fixed. In another example, presence or absence of restaurant can be calculated as the second characteristic amount from location data that is acquired from a location sensor, such as a GPS receiver or a wireless LAN access point. Presence or absence of restaurant is defined as the second characteristic amount as described above because there is a characteristic that places to drink alcohol drinking are roughly fixed. In another example, presence or absence of eating can be calculated as the second characteristic amount. Presence or absence of eating is defined as the second characteristic amount as described above because there is a characteristic that alcohol drinking and eating are often performed together.

(5) Sitting down

For example, when sitting-down sensing is performed, an amount of the following characteristic can be calculated as the first characteristic amount. For example, a change of decrease in the heart rate can be calculated as the first characteristic amount from heart rate data that is acquired from a heart rate sensor. A change of decrease is defined as the first characteristic amount because there is a characteristic that the heart rate tends to decrease in sitting down. In another example, a change in the posture for stretching or bending can be calculated as the first characteristic amount from motion data that is acquired from a motion sensor. A change in the posture is defined as a first characteristic amount because there is a characteristic that bending and stretching of the knees occur to sit down. In a still another example, presence or absence of operation of muscles of legs can be calculated as the first characteristic amount from myoelectric signals that are acquired from a myoelectric sensor. Presence or absence of operation of muscles is defined as the first characteristic amount because there is a characteristic that pressure is applied to the muscles of the legs to sit down.
Furthermore, when sitting-down sensing is performed, an amount of the following characteristic can be calculated as the second characteristic amount. In an example, presence or absence of walking can be calculated as the second characteristic amount from motion data that is acquired from a motion sensor. Presence or absence of walking is defined as the second characteristic amount as described above because there is a characteristic that walking is often performed after standing up.

(6) Standing up

For example, when standing-up sensing is performed, an amount of the following characteristic can be calculated as the first characteristic amount. For example, a change of increase in the heart rate can be calculated as the first characteristic amount from heart rate data that is acquired from a heart rate sensor. A change of increase is defined as the first characteristic amount as described above because there is a characteristic that the heart rate tends to increase after standing up compared to sitting down and lying down. In another example, a change in the posture for stretching or bending can be calculated as the first characteristic amount from motion data that is acquired from a motion sensor. A change in the posture is defined as the first characteristic amount because there is a characteristic that bending and stretching of the knees occur to stand up.
Furthermore, when standing-up sensing is performed, an amount of the following characteristic can be calculated as the second characteristic amount. In an example, presence or absence of walking can be calculated as the second characteristic amount from motion data that is acquired from a motion sensor. Presence or absence of walking is defined as the second characteristic amount as described above because there is a characteristic that walking is often performed after standing up.

(7) Walking

For example, when walking sensing is performed, an amount of the following characteristic can be calculated as a first characteristic amount. For example, presence or absence of periodic motion of the leg part can be calculated as the first characteristic amount from motion data that is acquired from a motion sensor. Presence or absence of periodic motion of the leg part is defined as the first characteristic amount because there is a characteristic that the left and right legs are moved alternately to walk. In another example, a change of increase in the heart rate can be calculated as the first characteristic amount from heart rate data that is acquired from a heart rate sensor. A change of increase is defined as the first characteristic amount as described above because there is a characteristic that the heart rate tends to increase in walking compared to sitting down and lying down.

Furthermore, when walking sensing is performed, an amount of the following characteristic can be calculated as the second characteristic amount. In an example, presence or absence of standing-up motion can be calculated as the second characteristic amount. Presence or absence of standing-up motion is defined as the second characteristic amount as described above because there is a characteristic that walking is often performed after standing up. In another example, presence or absence of an operation on a switch of a remote controller can be calculated as the second characteristic amount. It is defined as the second characteristic amount because there is a characteristic that walking tends to be performed to operate an appliance.

(8) Ascending and Descending

For example, when sensing of ascending and descending of stairs, or the like, is performed, an amount of the following characteristic can be calculated as the first characteristic amount. For example, presence or absence of periodic motion of the leg part can be calculated as the first characteristic amount from motion data that is acquired from a motion sensor. Presence or absence of periodic motion of the leg part is defined as the first characteristic amount because there is a characteristic that the left and right legs are moved alternately to walk. In another example, a change of increase in the heart rate can be calculated as the first characteristic amount from heart rate data that is acquired from a heart rate sensor. A change of increase is defined as the first characteristic amount as described above because there is a characteristic that the heart rate tends to increase during walking compared to sitting down and lying down. In still another example, presence or absence of shallow breaths and sweating can be calculated as the first characteristic amount. They are defined as the first characteristic amount because there is a characteristic that breaths tend to be short and sweating tends to occur during ascending and descending.

Furthermore, when ascending and descending sensing is performed, an amount of the following characteristic can be calculated as the second characteristic amount. In an example, a change in the pressure can be calculated as the second characteristic amount from atmospheric data that is acquired from an atmospheric sensor. A change in the atmosphere is defined as the second characteristic amount because there is a characteristic that descending and ascending are often associated with a change in the surrounding atmosphere.

(9) Sleeping

When sleep sensing is performed, an amount of the following characteristic can be calculated as the first characteristic amount. For example, a change in the heart rate, fluctuations in the heart rate (LF/HF), etc., can be calculated as the first characteristic amount from heart rate data that is acquired from a heart rate sensor. They are defined as the first characteristic amount because there is a characteristic that the heart rate during sleeping is lower than the heart rate in daytime and fluctuations in the heart rate are repeated between rapid eye movement sleep and a non-rapid eye movement sleep. In another example, eye movement can be calculated as the first characteristic amount. Eye movement is calculated as the first characteristic amount as described above because there is a characteristic that eye movement differs between rapid eye movement sleep and non-rapid eye movement sleep and eye moves hastily and stably repeatedly.

When sleep sensing is performed, an amount of the following characteristic can be calculated as the second characteristic amount. For example, presence or absence in a given place, such as home or a hotel, can be calculated as the second characteristic amount from location data that is acquired by a location sensor. Presence or absence in a given place is defined as the second characteristic amount as described above because there is a characteristic that a place for sleep is roughly fixed. In another example, the OFF mode of light can be calculated as the second characteristic amount from illuminance data that is acquired by an illuminance sensor. The OFF mode of light is defined as the second characteristic amount as described above because there is a characteristic that the light is highly likely to be turned off for sleep. In a still another example, presence or absence of sound can be calculated as the second characteristic amount from sound data that is acquired by a microphone. Presence or absence of sound is defined as the second characteristic amount because there is a characteristic that sleep is likely to be taken in a silent environment.

Stand Alone

Exemplary Embodiment 1 described above exemplifies the case where the system is structured as a client server system including the sensor terminal 10 and the server device 100; however, the embodiments are not limited thereto. For example, the sensor terminal 10, the server device 100 or another computer may be caused to execute the series of processes from acquisition of sensor data to behavior sensing in a stand-alone manner.

Exemplary Application of System

In Exemplary Embodiment 1, the healthcare support system 1 includes the server device 100 but the healthcare support system 1 need not necessarily include the server device 100. In other words, when the sensor terminal 10 is mounted as a wearable gadget, or the like, a smartphone or a tablet terminal that is connected by near field communication using the wearable gadget may be caused to execute various processes other than acquisition of sensor data, such as behavior sensing.

Distribution and Integration

Each component of each device illustrated in the drawings need not necessarily be configured physically as illustrated in the drawings. In other words, specific modes of dispersion or integration in each device is not limited to those illustrated in the drawings and all or part of the components may be distributed or integrated functionally or physically according to a given unit in accordance with various types of load and usage. For example, the acquisition unit 120, the first characteristic amount calculator 130A, the second characteristic amount calculator 130B, the first probability calculator 140A, the second probability calculator 140B, the corrector 150 and the determination unit 160 may be connected via a network as an external device of the information processing device 100. Different devices may respectively include the acquisition unit 120, the first characteristic amount calculator 130A, the second characteristic amount calculator 130B, the first probability calculator 140A, the second probability calculator 140B, the corrector 150 and the determination unit 160 and may be connected via a network to cooperate to implement the functions of the above-described information processing device 100.

Behavior Sensing Program

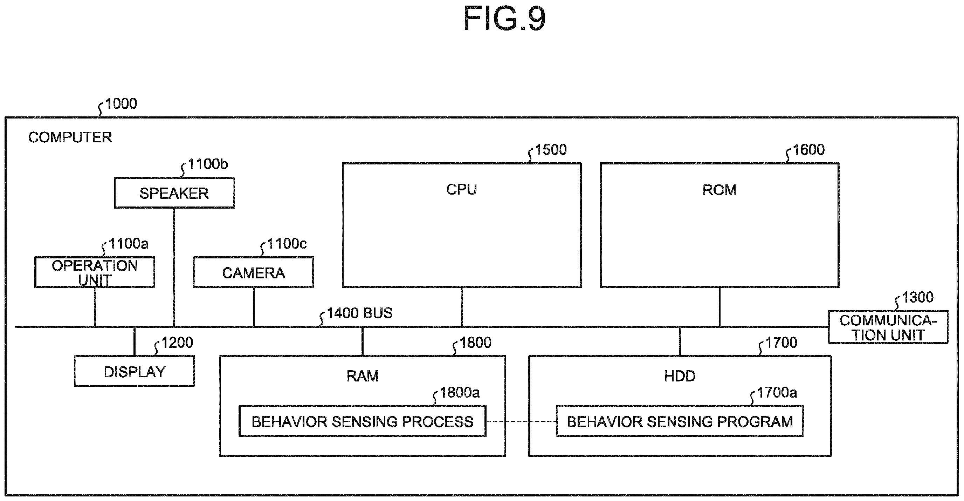

The various processes illustrated in the above-described exemplary embodiments can be implemented by executing a program that is prepared in advance with a computer, such as a personal computer or a work station. An exemplary computer that executes the behavior sensing program having the same functions as those of the above-described exemplary embodiments will be described below using FIG. 9.
FIG. 9 is a diagram illustrating an exemplary hardware configuration of a computer that executes a behavior sensing program according to Exemplary Embodiment 1 and Exemplary Embodiment 2. As illustrated in FIG. 9, a computer 1000 includes an operation unit 1100a, a speaker 1100b, a camera 1100c, a display 1200 and a communication unit 1300. The computer 1000 further includes a CPU 1500, a ROM 1600, a HDD 1700 and a RAM 1800. The components 1100 to 1800 are connected with one another via a bus 1400.
As illustrated in FIG. 9, a behavior sensing program 1700a that implements the same functions as those of the acquisition unit 120, the first characteristic amount calculator 130A, the second characteristic amount calculator 130B, the first probability calculator 140A, the second probability calculator 140B, the corrector 150 and the determination unit 160 is stored in the HDD 1700. Integration or separation of the behavior sensing program 1700a may be implemented as in the case of the components that are the acquisition unit 120, the first characteristic amount calculator 130A, the second characteristic amount calculator 130B, the first probability calculator 140A, the second probability calculator 140B, the corrector 150 and the determination unit 160. In other words, all the data represented in Exemplary Embodiment 1 described above need not necessarily be stored in the HDD 1700 and it suffices if data to be used for processes is stored in the HDD 1700.
Under such a circumstance, the CPU 1500 reads the behavior sensing program 1700a from the HDD 1700 and then loads the behavior sensing program 1700a into the RAM 1800. As a result, as illustrated in FIG. 9, the behavior sensing program 1700a functions as a behavior sensing process 1800a. The behavior sensing process 1800a loads various types of data that is read from the HDD 1700 into an area that is allocated to the behavior sensing process 1800a in a storage area of the RAM 1800 and executes various processes using the loaded various types of data. For example, exemplary processes executed by the behavior sensing process 1800a include the processes illustrated in FIGS. 7 and 8. In the CPU 1500, all the processes represented in Exemplary Embodiment 1 need not necessarily operate, and it suffices if a processor corresponding to a process to be executed is implemented virtually.

The above-described behavior sensing program 1700a need not necessarily be stored in the HDD 1700 or the ROM 1600 from the beginning. For example, each program is stored in a "portable physical medium", such as flexible disk, that is, a FD, a CD-ROM, a DVD disk, a magnetooptical disk or an IC card that is inserted into the computer 1000. The computer 1000 may acquire each program from the portable physical medium and execute the program. Each program may be stored in another computer or a server device that is connected to the computer 1000 via a public line, the Internet, a LAN or a WAN and the computer 1000 may acquire each program from them and execute the program.

[Explanation of Reference]

| | |
|---|---|
| 1 | HEALTHCARE SUPPORT SYSTEM |
| 20 | SENSOR TERMINAL |
| 11 | SENSOR DATA ACQUISITION UNIT |
| 13 | COMMUNICATION I/F UNIT |
| 100 | INFORMATION PROCESSING DEVICE |
| 110 | COMMUNICATION I/F UNIT |
| 120 | ACQUISITION UNIT |
| 130A | FIRST CHARACTERISTIC AMOUNT CALCULATOR |
| 130B | SECOND CHARACTERISTIC AMOUNT CALCULATOR |
| 140A | FIRST PROBABILITY CALCULATOR |
| 140B | SECOND PROBABILITY CALCULATOR |
| 150 | CORRECTOR |
| 160 | DETERMINATION UNIT |

**Claims**

1. A behavior sensing device comprising:

   an acquisition unit configured to acquire sensor data from one or more sensors;
   a first characteristic amount calculator configured to calculate a first characteristic amount serving as an index of characteristic of biological reaction to a behavior to be sensed from the sensor data;
   a first probability calculator configured to calculate a probability of occurrence of the behavior as a first behavior occurrence probability from the first characteristic amount;
   a second characteristic amount calculator configured to calculate a second characteristic amount serving as an index of characteristic of a habit relating to the behavior to be sensed from the sensor data;
   a second probability calculator configured to calculate a probability of occurrence of the behavior as a second behavior occurrence probability from the second characteristic amount; and
   a determination unit configured to determine whether the behavior is performed based on the first behavior occurrence probability and the second behavior occurrence probability.

2. The behavior sensing device according to claim 1, further comprising a corrector configured to correct a weight of at least any one of the first behavior occurrence probability and the second behavior occurrence probability, wherein the determination unit is configured to determine whether the behavior is performed based on the weight of at least any one of the corrected first behavior occurrence probability and the corrected second behavior occurrence probability.

3. The behavior sensing device according to claim 2, wherein the corrector performs correction to reduce the weight of the second behavior occurrence probability to one smaller than the weight of the first behavior occurrence probability.

4. The behavior sensing device according to claim 2, wherein, when a value of the first behavior occurrence probability is larger than a given value, the corrector performs correction to reduce the weight of the second behavior occurrence probability.

5. The behavior sensing device according to claim 2, wherein, when a value of the first behavior occurrence probability is smaller than a given value, the corrector performs correction to reduce the weight of the second behavior occurrence probability.

6. A behavior sensing method, wherein a computer executes processes of
acquiring sensor data from one or more sensors;
calculating a first characteristic amount serving as an index of characteristic of biological reaction to a behavior to be sensed from the sensor data;
calculating a probability of occurrence of the behavior as a first behavior occurrence probability from the first characteristic amount;
calculating a second characteristic amount serving as an index of characteristic of a habit relating to the behavior to be sensed from the sensor data;
calculating a probability of occurrence of the behavior as a second behavior occurrence probability from the second characteristic amount; and
determining whether the behavior is performed based on the first behavior occurrence probability and the second behavior occurrence probability.

7. The behavior sensing method according to claim 6, wherein the computer further executes a process of correcting a weight of at least any one of the first behavior occurrence probability and the second behavior occurrence probability, wherein the process of determining includes determining whether the behavior is performed based on the weight of at least any one of the corrected first behavior occurrence probability and the corrected second behavior occurrence probability.

8. The behavior sensing method according to claim 7, wherein the process of correcting includes performing correction to reduce the weight of the second behavior occurrence probability to one smaller than the weight of the first behavior occurrence probability.

9. The behavior sensing method according to claim 7, wherein the process of correcting includes, when a value of the first behavior occurrence probability is larger than a given value, performing correction to reduce the weight of the second behavior occurrence probability.

10. The behavior sensing method according to claim 7, wherein the process of correcting includes, when a value of the first behavior occurrence probability is smaller than a given value, performing correction to reduce the weight of the second behavior occurrence probability.

11. A behavior sensing program that causes a computer to execute a process comprising:

acquiring sensor data from one or more sensors;
calculating a first characteristic amount serving as an index of characteristic of biological reaction to a behavior to be sensed from the sensor data;
calculating a probability of occurrence of the behavior as a first behavior occurrence probability from the first characteristic amount;
calculating a second characteristic amount serving as an index of characteristic of a habit relating to the behavior to be sensed from the sensor data;
calculating a probability of occurrence of the behavior as a second behavior occurrence probability from the second characteristic amount; and
determining whether the behavior is performed based on the first behavior occurrence probability and the second behavior occurrence probability.

12. The behavior sensing program according to claim 11, further causing the computer to executes a process of correcting a weight of at least any one of the first behavior occurrence probability and the second behavior occurrence probability, wherein the process of determining includes determining whether the behavior is performed based on the weight of at least any one of the corrected first behavior occurrence probability and the corrected second behavior occurrence probability.

13. The behavior sensing program according to claim 12, wherein the process of correcting includes performing correction to reduce the weight of the second behavior occurrence probability to one smaller than the weight of the first behavior occurrence probability.

14. The behavior sensing program according to claim 12, wherein the process of correcting includes, when a value of the first behavior occurrence probability is larger than a given value, performing correction to reduce the weight of the second behavior occurrence probability.

15. The behavior sensing program according to claim 12, wherein the process of correcting includes, when a value of the first behavior occurrence probability is smaller than a given value, performing correction to reduce the weight of the second behavior occurrence probability.

# FIG.1

EP 3 376 462 A1

FIG.2

FIG.3

# FIG.4

SECOND BEHAVIOR OCCURRENCE PROBABILITY

SENSED

NOT SENSED

30

FIRST BEHAVIOR OCCURRENCE PROBABILITY

# FIG.5

FIRST BEHAVIOR OCCURRENCE PROBABILITY

SECOND CHARACTERISTIC AMOUNT

FIRST CHARACTERISTIC AMOUNT

SECOND BEHAVIOR OCCURRENCE PROBABILITY

# FIG.6

EP 3 376 462 A1

# FIG.7

```
                    START

                                    S101
            ACQUIRE TEACHING DATA

                                    S102
        ACQUIRE ONE OR MORE SETS OF SENSOR DATA


         S103A                              S103B
CALCULATE FIRST CHARACTERISTIC AMOUNT   CALCULATE SECOND CHARACTERISTIC AMOUNT

         S104A                              S104B
   GENERATE FIRST LEARNING DATA BY         GENERATE SECOND LEARNING DATA BY
ASSOCIATING FIRST CHARACTERISTIC AMOUNT   ASSOCIATING SECOND CHARACTERISTIC
AND TEACHING DATA WITH EACH OTHER       AMOUNT AND TEACHING DATA WITH EACH OTHER

         S105A                              S105B
GENERATE FIRST DETERMINATION MODEL      GENERATE SECOND DETERMINATION MODEL

         S106A                              S106B
CALCULATE FIRST BEHAVIOR OCCURRENCE     CALCULATE SECOND BEHAVIOR OCCURRENCE
           PROBABILITY                            PROBABILITY

         S107
CORRECT FIRST BEHAVIOR OCCURRENCE
           PROBABILITY


                                    S108
         ASSOCIATE PROBABILITIES AT THE SAME TIME

                                    S109
          GENERATE INTEGRATED LEANING DATA
           BY ASSOCIATING PROBABILITY AND
           TEACHING DATA WITH EACH OTHER

                                    S110
        GENERATE INTEGRATED DETERMINATION MODEL


                    END
```

# FIG.8

```
                    ┌──────────────────┐
                    │      START       │
                    └──────────────────┘
                              │
                              ▼              ╭S102
              ┌───────────────────────────────────┐
              │  ACQUIRE ONE OR MORE SETS OF      │
              │         SENSOR DATA               │
              └───────────────────────────────────┘
                              │
            ┌─────────────────┴─────────────────────────┐
            ▼          ╭S103A                            ▼          ╭S103B
 ┌───────────────────────────────┐        ┌───────────────────────────────────┐
 │ CALCULATE FIRST CHARACTERISTIC│        │ CALCULATE SECOND CHARACTERISTIC   │
 │           AMOUNT              │        │            AMOUNT                 │
 └───────────────────────────────┘        └───────────────────────────────────┘
            │          ╭S106A                            │          ╭S106B
            ▼                                            ▼
 ┌───────────────────────────────┐        ┌───────────────────────────────────┐
 │    CALCULATE FIRST BEHAVIOR   │        │    CALCULATE SECOND BEHAVIOR      │
 │    OCCURRENCE PROBABILITY     │        │    OCCURRENCE PROBABILITY         │
 └───────────────────────────────┘        └───────────────────────────────────┘
            │          ╭S107                             │
            ▼                                            │
 ┌───────────────────────────────┐                      │
 │    CORRECT FIRST BEHAVIOR     │                      │
 │    OCCURRENCE PROBABILITY     │                      │
 └───────────────────────────────┘                      │
            │                                            │
            └─────────────────┬──────────────────────────┘
                              ▼              ╭S108
              ┌───────────────────────────────────┐
              │  ASSOCIATE PROBABILITIES AT THE   │
              │            SAME TIME              │
              └───────────────────────────────────┘
                              │              ╭S201
                              ▼
              ┌───────────────────────────────────┐
              │  BEHAVIOR SENSING BASED ON        │
              │       INTEGRATED MODEL            │
              └───────────────────────────────────┘
                              │
                              ▼
                    ┌──────────────────┐
                    │       END        │
                    └──────────────────┘
```

# FIG.9

## INTERNATIONAL SEARCH REPORT

| International application No. |
| --- |
| PCT/JP2015/082050 |

A. CLASSIFICATION OF SUBJECT MATTER
*G06Q50/10(2012.01)i*

According to International Patent Classification (IPC) or to both national classification and IPC

B. FIELDS SEARCHED

Minimum documentation searched (classification system followed by classification symbols)
G06Q50/10

Documentation searched other than minimum documentation to the extent that such documents are included in the fields searched
Jitsuyo Shinan Koho        1922–1996   Jitsuyo Shinan Toroku Koho   1996–2016
Kokai Jitsuyo Shinan Koho  1971–2016   Toroku Jitsuyo Shinan Koho   1994–2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2015-154889 A  (Seiko Epson Corp.), 27 August 2015 (27.08.2015), paragraphs [0021] to [0037], [0048] (Family: none) | 1-15 |
| Y | JP 2013-3649 A  (Sony Corp.), 07 January 2013 (07.01.2013), paragraphs [0024], [0156] to [0162] & US 2014/0101169 A1 paragraphs [0109], [0262] to [0270] & US 2016/026349 A1      & WO 2012/173027 A1 & EP 2720176 A1          & CN 103597476 A | 1-15 |

☐ Further documents are listed in the continuation of Box C.        ☐ See patent family annex.

| | |
| --- | --- |
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 02 February 2016 (02.02.16) | 09 February 2016 (09.02.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**EP 3 376 462 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013109623 A